(19) Europäisches Patentamt
European Patent Office
Office européen des brevets

(11) **EP 4 600 342 A1**

(12) **EUROPEAN PATENT APPLICATION**
published in accordance with Art. 153(4) EPC

(43) Date of publication:
**13.08.2025 Bulletin 2025/33**

(21) Application number: **23875173.9**

(22) Date of filing: **27.09.2023**

(51) International Patent Classification (IPC):
***C12M 1/42*** *(2006.01)*    ***C12M 3/00*** *(2006.01)*
***C12M 1/00*** *(2006.01)*    ***C12N 15/87*** *(2006.01)*

(52) Cooperative Patent Classification (CPC):
**C12N 13/00; C12M 23/42; C12M 27/00;
C12M 35/02; C12N 15/87**

(86) International application number:
**PCT/KR2023/015094**

(87) International publication number:
**WO 2024/076109 (11.04.2024 Gazette 2024/15)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.10.2022 KR 20220128485**

(71) Applicant: **Femtobiomed Inc.
Seongnam-si, Gyeonggi-do 13516 (KR)**

(72) Inventors:
• **LEE, Sanghyun
Seongnam-si, Gyeonggi-do 13516 (KR)**
• **CHOI, Juhyun
Seoul 05849 (KR)**
• **PARK, Jun Kwon
Yongin-si, Gyeonggi-do 17007 (KR)**

(74) Representative: **Ipsilon Lyon
Le Contemporain
50 Chemin de la Bruyère
69574 Dardilly Cedex (FR)**

(54) **TRANSFORMATION CARTRIDGE, TRANSFORMATION DEVICE, TRANSFORMATION SYSTEM COMPRISING SAME, AND TRANSFORMATION METHOD USING SAME**

(57) A cell transformation cartridge according to the present invention includes a body unit, in which a cell flow path, a material flow path, and a resultant flow path that meet one another are formed; and an electric field forming unit, which includes an electrode being connected to the body unit to create an electric field in a resultant flow path, wherein the cell flow path and the material flow path are formed in a shape that converges to the resultant flow path, wherein the resultant flow path includes a mixing flow path extending from a location being connected to the cell flow path and the material flow path, and wherein the thickness of the mixing flow path is smaller than that of the cell flow path.

FIG.4

## Description

### TECHNICAL FIELD

The present invention relates to a transformation cartridge, a transformation device, a transformation system including the same, and a transformation method using the same.

### BACKGROUND ART

As gene transfer technologies for transformation, a viral vector-based transfer method using a virus as a transporter and a non-viral delivery technique using a synthetic phospholipid or a synthetic cationic polymer, etc. are widely known.

Viruses can be regarded as a very efficient DNA delivery system because they live as a parasite in the state that their DNA is inserted into the nucleus of other cells due to their own life mechanisms. That is, in a DNA delivery system using a virus, the gene to be delivered is inserted into a non-replicable virus to be used. The advantage of this technique lies in that it is very efficient for transformation. However, this technique has several drawbacks as follows: from an essential point of view, it has a problem in that it is possible to generate a recombination competent virus (RCV) even though an inactivated virus is used and a problem in that it is difficult to use the virus multiple times because it causes an immune response; a problem in that the materials that can be delivered by the virus is limited by its intrinsic properties; a problem in that it is difficult to inject a certain amount of material into the cells, *etc.*

In order not to use a viral vector, a method that opens the phospholipid double membrane by giving an electric shock in a state where a material is located around the cell (electroporation) may be used. However, during general electroporation, there occurs a situation in which the cells should be placed in a special buffer (e.g., RNase-free buffer) rather than in a culture medium due to the nature of the material to be delivered, and to this end, there is a need of pretreatment of the culture solution, which is a process of washing the cells by centrifugation, etc.

When such a typical electroporation method is used, cell viability may be significantly reduced, and there is a problem in that a large amount of cell loss may occur during the pretreatment process. In addition, there is a problem in that the special buffer used is very expensive and not economical. In addition, there is a problem in that it is difficult to perform tasks such as pretreatment in a closed environment; therefore, it is difficult to perform automation and it is difficult to hygienically control the working environment.

### DISCLOSURE OF THE INVENTION

### TECHNICAL PROBLEM

The present invention, which was devised to solve the problems described above, provides a cell transformation cartridge that allows transformation to occur only by electroporation alone by adding a cell solution and a material solution, a cell transformation device, a cell transformation system including the same, and a cell transformation method using the same.

### TECHNICAL SOLUTION

According to an embodiment of the present invention, the cell transformation cartridge includes: a body unit, in which a cell flow path, a material flow path, and a resultant flow path that meet one another are formed; and an electric field forming unit, which includes an electrode being connected to the body unit to create an electric field in a resultant flow path, wherein the cell flow path and the material flow path are formed in a shape that converges to the resultant flow path, wherein the resultant flow path includes a mixing flow path extending from a location being connected to the cell flow path and the material flow path, and wherein the thickness of the mixing flow path is smaller than that of the cell flow path.

According to an embodiment of the present invention, the cell transformation cartridge includes: a body unit, in which a cell flow path, a material flow path, and a resultant flow path that meet one another are formed; and an electric field forming unit, which includes an electrode that is connected to the body unit and is placed at an inlet of the material flow path and an outlet of the resultant flow path; wherein the cell flow path and the material flow path are formed in a shape that converges to the resultant flow path, wherein the resultant flow path includes a mixing flow path extending from a location being connected to the cell flow path and the material flow path, and wherein the value obtained by multiplying (the thickness of the mixing flow path) and (the value obtained by dividing the cell flow path by the sum of the thickness of the cell flow path and the thickness of the material flow path) is smaller than the diameter of the cells flowing in the cell flow path.

According to an embodiment of the present invention, the cell transformation device includes: a pump unit, which is provided for pressurized supply of a cell solution and a material solution to a cell flow path and a material flow path of a cell transformation cartridge, respectively; a power application unit, which is provided to be connected to a terminal of the cell transformation cartridge to apply power to an electrode of the cell transformation cartridge; and a processor, which is electrically connected to the power application unit and the pump unit, wherein the processor controls the pump unit based on the information of the cell transformation cartridge, a preset flow ratio, and a preset exposure time.

According to an embodiment of the present invention, the cell transformation system includes: a body unit, in which a cell flow path, a material flow path, and a resultant flow path that meet one another are formed; an electric field forming unit, which includes an electrode that is connected to the body unit and is placed at an inlet of the material flow path and an outlet of the resultant flow path; a pump unit, which is provided for pressurized supply of a cell solution and a material solution to the cell flow path and the material flow path, respectively; and a processor, which is electrically connected to the pump unit, wherein the cell flow path and the material flow path are formed in a shape that converges to the resultant flow path, wherein the resultant flow path includes a mixing flow path extending from a location being connected to the cell flow path and the material flow path, and the processor controls the pump unit such that the thickness of the solution being flowed into the mixing flow path through the cell flow path within the mixing flow path is smaller than the diameter of the cells contained in the cell solution.

According to an embodiment of the present invention, the cell transformation method includes: preparing a cartridge, in which a cell flow path, a material flow path, and a mixing flow path that meet one another are formed; injecting a cell solution comprising cells into the cell flow path; and injecting a material solution comprising a material for transformation of the cells into the material flow path, wherein the ratio between the flow rate of the cell solution and the flow rate of the material solution being injected is a value that allows transformation to occur when the cells and the material meet in the mixing flow path.

## ADVANTAGEOUS EFFECTS

Accordingly, transformation can occur by electroporation merely by injecting a cell solution and a material solution.

The transformation can occur even if the cell solution and the material solution and are not mixed.

Since pretreatment such as cell washing can be omitted for cells or transformants, cell viability can be increased during the corresponding process, and the loss in cells and materials can be significantly reduced.

Since the use of a separate special buffer is not required, it is possible to constitute an economical transformation process.

Since pretreatment is not required, the transformation process can be constituted merely by enabling the injection of the solution into a closed and controlled environment; therefore, a sanitary working environment can be maintained and is advantageous from various regulations.

Since all necessary processes for the transformation are merely injecting a culture medium containing cells and a material solution and applying an electric field are, the automation of the processes can easily be achieved

## BRIEF DESCRIPTION OF THE DRAWINGS

FIG. 1 is a perspective view of a cell transformation cartridge according to the first embodiment of the present invention.
FIG. 2 is an exploded perspective view of a cell transformation cartridge according to the first embodiment of the present invention.
FIG. 3 is a plan view of a cell transformation cartridge according to the first embodiment of the present invention.
FIG. 4 is a conceptual diagram of a body unit of a cell transformation cartridge according to the first embodiment of the present invention.
FIG. 5 is a conceptual diagram of a body unit of a cell transformation cartridge according to the second embodiment of the present invention.
FIG. 6 is a conceptual diagram of a body unit of a cell transformation cartridge according to the third embodiment of the present invention.
FIG. 7 is a perspective view of a cell transformation cartridge according to the fourth embodiment of the present invention.
FIG. 8 is an exploded perspective view of a cell transformation cartridge according to the fourth embodiment of the present invention.
FIG. 9 is a view showing a cell transformation system including a cell transformation cartridge and a cell transformation device according to the first embodiment of the present invention.
FIG. 10 is a view showing a cell transformation system including a cell transformation cartridge and a cell transformation device according to the fourth embodiment of the present invention.
FIG. 11 is a view showing the settlement of cells occurring in a cell transformation cartridge according to the first embodiment of the present invention.
FIG. 12 is a view showing the settlement of cells occurring in a cell transformation cartridge according to the third embodiment of the present invention.
FIG. 13 shows the experimental results confirming the mRNA transformation efficiency using the Neon™ Transfection System (ThermoFisher) and the mRNA transformation efficiency using a cell transformation cartridge of the present invention.
FIG. 14 shows the experimental results, during the process of performing transformation by separately supplying a cell solution and a material solution using a cell transformation cartridge of the present invention, confirming the effect of the ratio of flow rates between the two solutions in the mixing flow path on the delivery efficiency of materials such as mRNA.
FIG. 15 shows the experimental results in the case of using a cell transformation cartridge of the present invention, confirming by repeating three times to determine whether materials can be delivered with high efficiency even to the cells collected from the human body (primary cells).

FIG. 16 shows the experimental results confirming the construction efficiency (transduction efficiency) of CAR-NK cells constructed using the cell transformation cartridge of the present invention (A) and the degree of killing of cancer cells (B).

## MODE FOR CARRYING OUT THE INVENTION

Exemplary embodiments of the present invention will now be described in detail with reference to the accompanying drawings. In adding reference numerals to the components of each drawing, it should be noted that the same components are given the same reference numerals as much as possible even though they are indicated on different drawings. In addition, in describing an embodiment of the present invention, if it is determined that a detailed description of a related known constitution or function interferes with the understanding of the embodiment of the present invention, the detailed description will be omitted.

In addition, in describing the components of the embodiments of the present invention, terms such as first, second, A, B, (a), and (b) may be used. These terms are only for distinguishing the components from other components, and the essence, order, sequence, etc. of the components are not limited by these terms. When a component is described as "connected", "coupled" or "conjugated" to another element, the component may be directly connected or conjugated to the another component, but it should be understood that still another component may be "connected", "coupled" or "conjugated" between each component.

## First embodiment

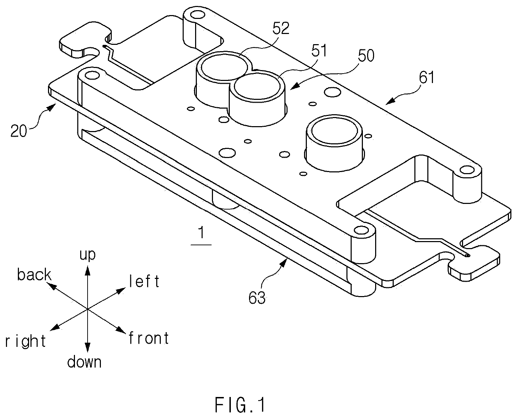

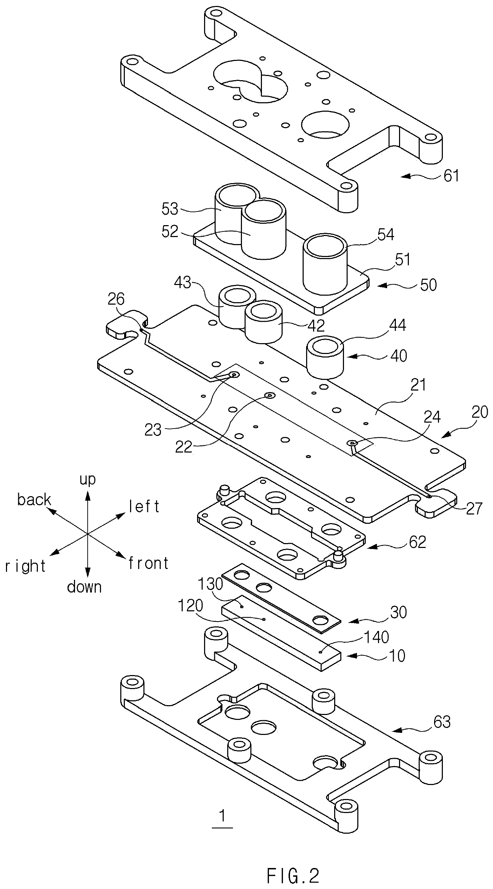

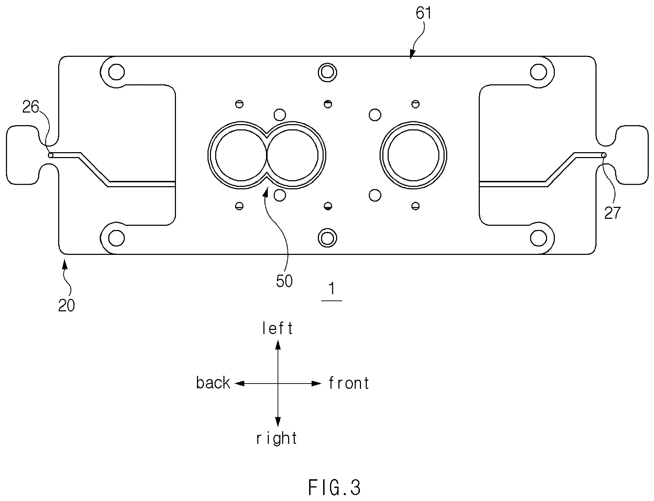

FIG. 1 is a perspective view of a cell transformation cartridge 1 according to the first embodiment of the present invention. FIG. 2 is an exploded perspective view of a cell transformation cartridge 1 according to the first embodiment of the present invention. FIG. 3 is a plan view of a cell transformation cartridge 1 according to the fFirst embodiment of the present invention.

A cell transformation cartridge 1 according to the first embodiment of the present invention includes a body unit 10 and an electric field forming unit 20. In the specification of the present invention, the up and down, left and right, front and back directions are orthogonal to each other used for convenience of explanation, and is a relative direction that can be changed according to the arrangement state of the cell transformation cartridge 1.

An electric field forming unit 20 is a component that forms an electric field in a body unit 10. The electric field forming unit 20 includes an electrode which is connected to a body unit 10 and disposed at an inlet 130 of a material flow path 13 and an outlet 140 of a resultant flow path 14 so as to create an electric field in a resultant flow path 14. The electric field forming unit 20 may be a printed circuit board, but its type is not limited thereto.

The electric field forming unit 20 may include a plate-shaped electric field forming body 21. The plate-shaped electric field forming body 21 may consist of a multi-layered epoxy resin, but the material thereof is not limited thereto. An electrode may be formed in the electric field forming body 21, and each electrode may be formed in an annular shape to surround each hole formed in the electric field forming unit 20 so as to correspond to each flow path of the body unit 10.

The electrodes (22, 23, 24) may be formed of a conductor such as a metal. The electrode may be formed by coating copper on the electric field forming body 21. The electrode of the electric field forming unit 20 may include a cell electrode 22, a material electrode 23, and a resultant electrode 24. The electric field forming unit 20 may include terminals (26, 27), which can be electrically connected to each of the electrodes (22, 23, 24) and capable of being connected to a power source. The terminals (26, 27) may be formed of the same material as the electrodes (22, 23, 24). The cell transformation cartridge 1 according to the first embodiment of the present invention may include a material terminal 26 electrically connected to the material electrode 23 and a resultant terminal 27 electrically connected to the resultant electrode 24. Each of the terminals (26, 27) may be disposed on the outside of the electric field forming body 21 so as to be easily connected to the power source.

The electric field forming unit may be configured to include electrodes arranged to surround each flow path inside the body unit and coupled to the body unit. These electrodes may also be placed in a mixing flow path.

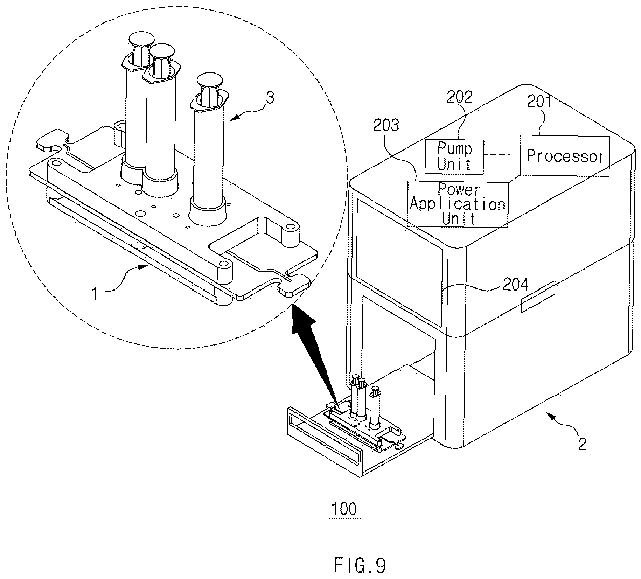

The electric field forming unit 20 may form an electric field using a simple DC power source, but may also form an electric field using a pulse-type power source. The processor 201 of a cell transformation device (2 of FIG. 9), to be described later, can control the electric field forming unit 20 in a PWM control manner using a power application unit 203 electrically connected thereto. The frequency of the pulse may be 1 kHz or more and 100 kHz or less, and the duty ratio may be 10% or more and 90% or less. The electric field forming unit 20 may be formed while changing the size of the electric field. The size of the electric field can be changed by being reduced up to 50% of the maximum value of the size of the electric field formed by the electric field forming unit 20.

The cell transformation cartridge 1 according to the first embodiment of the present invention may include a sealing sheet 30. The sealing sheet 30 is located between the body unit 10 and a lower mount 63 to be described later, and thus can prevent an impact from being directly transmitted from the lower mount 63 to the body unit 10 and to stably support the body unit 10. The sealing sheet 30 may be formed of a material including silicon, but the material is not limited thereto. A hole is formed in a location on the sealing sheet 30 corresponding to the lower side of the inlet of each flow path (12, 13, 14), so that the state of each flow path (12, 13, 14) can be checked from the lower side to the upper side.

The cell transformation cartridge 1 according to the first embodiment of the present invention may include an O-ring unit 40. The O-ring unit 40 consists of annular O-rings (42, 43, 44) having elasticity, is located between the electric field forming unit 20 and the guide unit 50 to be described later, and thus can maintain water tightness between the electric field forming unit 20 and the guide unit 50, and can hold the needle being inserted into the guide unit 50 thereby capable of stably injecting the solution. The O-ring unit 40 may be formed of a material containing silicon, but the material is not limited thereto. The O-ring unit 40 may include a cell O-ring 42 located between the cell electrode 22 and the cell guide 52; a material O-ring 43 located between the material electrode 23 and the material guide 53; and a resultant O-ring 44 located between the resultant electrode 24 and the resultant guide 54.

The cell transformation cartridge 1 according to the first embodiment of the present invention may include a guide unit 50. The guide unit 50 may serve as an inlet and outlet for a solution. The guide unit 50 may include a plate-shaped guide body 51, a cell guide 52 coupled to the guide body 51, a material guide 53, and a resultant guide 54. Each of the guides (52, 53, 54) may be formed in a pipe shape extending upward from the guide body 51. Each of the guides (52, 53, 54) has a shape of vertical penetration and is disposed at a location corresponding to each of the electrodes (22, 23, 24) and the flow paths 12, 13, and 14; therefore, when the needle is inserted into the guides (52, 53, 54) and the liquid is discharged in such a way that it is connected to the pump, etc., thus enabling the delivery of the discharged liquid to each flow path through each electrode. A needle may be stably inserted into each of the guides (52, 53, 54) to enable the discharge of the liquid at an appropriate location. O-rings (42, 43, 44) may be disposed on the lower side of each of the guides (52, 53, 54); and an electric field forming unit 20 may be located on the lower side of the guide body 51. The guide unit 50 may be formed of a material including polycarbonate, but the material is not limited thereto.

The cell transformation cartridge 1 according to the first embodiment of the present invention may include an upper mount 61 and a lower mount 63. The upper mount 61 may be located above the guide unit 50, and the lower mount 63 may be located below the sealing sheet 30. The upper mount 61 and the lower mount 63 may be fastened to each other by fasteners such as bolts, thereby fixing the sealing sheet 30, the body unit 10, the electric field forming unit 20, the O-ring unit 40, and the guide unit 50 located therebetween in a stacked state. The components therebetween may be pressed by the upper mount 61 and the lower mount 63, so that sealing may occur effectively. A hole through which the guide can pass may be formed on the upper mount 61, and a hole may be formed on the lower mount 63 at a location corresponding to the hole of the sealing sheet 30. The upper mount 61 and the lower mount 63 may consist of a material containing aluminum, and the aluminum may be anodized aluminum, but the material thereof is not limited thereto.

The cell transformation cartridge 1 according to the first embodiment of the present invention may include a body mount 62. The body mount 62 may include a hole in the center thereof into which the body unit 10 is inserted, so that the body unit 10 can be aligned with other components while being placed at an appropriate location. The body mount 62 may be located between the upper mount 61 and the lower mount 63 and may be further coupled through a fastener. The body mount 62 may consist of a material containing aluminum, and the aluminum may be anodized aluminum, but the material thereof is not limited thereto.

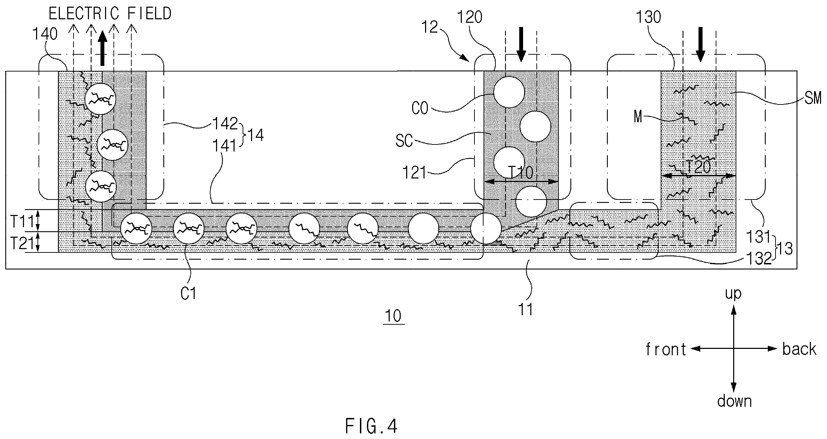

FIG. 4 is a conceptual diagram of a body unit 10 of a cell transformation cartridge 1 according to the first embodiment of the present invention.

A cell flow path 12, a material flow path 13, and a resultant flow path 14 meeting one another are formed in the body unit 10. These flow paths (12, 13, 14) may be formed through a body unit body 11. The body unit body 11 may be formed of a material including glass, and the glass may be borosilicate glass, but the material is not limited thereto.

An inlet (120, 130) or outlet 140 of each flow path (12, 13, 14) may be disposed on the upper surface of the body unit body 11. The inlet 130 of the material flow path 13, the inlet 120 of the cell flow path 12 and the outlet 140 of the resultant flow path 14 may be arranged in the order of the inlet 130 of the material flow path 13, the inlet 120 of the cell flow path 12 and the outlet 140 of the resultant flow path 14, as they go forward, which is the standard direction. A cell solution (SC) may be introduced into the inlet 120 of the cell flow path 12, a material solution SM may be introduced into the inlet 130 of a material flow path 13, and the resultant solution may be discharged to the outlet 140 of the resultant flow path 14.

The cell flow path 12 is a flow path through which the cell solution SC is introduced. The cell solution (SC) may consist of a culture medium containing cells (C0) or electrolyte. The cells (C0) included in the cell solution (SC) may be blood cells among somatic cells, and among them, may be immune cells. The immune cells may be used without limitation as long as they are cells capable of inducing immunity to induce a desired therapeutic effect, for example, any one selected from the group consisting of natural killer cells (NK cells), T cells, natural killer T cells (NKT cells), cytokine induced killer cells (CIK cells), macrophages, and dendritic cells, but are not limited thereto. The cell flow path 12 may include a cell introduction flow path 121 extending from the inlet 120 of the cell flow path 12. The cell introduction flow path 121 may extend downward from the inlet 120 of the cell flow path 12.

The material flow path 13 is a flow path through which the material solution (SM) is introduced and flows. The material solution (SM) may be water or a buffer containing a transformant (M). The transformant (M) may be one which is introduced into the inside of the cell (C0), for example, into the cytoplasm or nucleus to exert a function in the cell (C0), and is not limited to any form, such as a protein, a peptide, or a nucleic acid, as long as it is a material capable of exerting a function in the cell (C0). In particular, the transformant (M) may be a nucleic acid, and among others, mRNA. The material flow path 13 may include a material introduction flow path 131 extending from the inlet 130 of the material flow path 13. The material introduction flow path 131 may extend downward from the inlet 130 of the material flow path 13. The material flow path 13 may include a material transfer flow path 132 extending from the lower end of the material introduction flow path 131 toward the resultant flow path 14. The material transfer flow path 132 may extend forward in a horizontal direction. Therefore, the material flow path 13 may be bent at a location where the material introduction flow path 131 and the material transfer flow path 132 meet each other, and the bending angle may be 90 degrees.

The cell flow path 12 and the material flow path 13 may meet in a state where the cell flow path 12 is located on the upper side of the material flow path 13. The lower end of the cell introduction flow path 121 may be located on the upper side of the front end of the material transfer flow path 132. Therefore, the cell solution (SC) may be introduced into the resultant flow path 14 in a state located on the upper side of the material solution (SM), at a location where the material flow path 13 and the cell flow path 12 meet each other.

The resultant flow path 14 is a flow path through which the resultant solution containing the transformed cells (C1) flows. The resultant solution may be discharged through the outlet 140 of the resultant flow path 14. The cell flow path 12 and the material flow path 13 are formed to converge to the resultant flow path 14. Therefore, the cell solution (SC) and the material solution (SM) are combined to form the resulting solution.

The resultant flow path 14 includes a cell flow path 12 and a mixing flow path 141 extending from a location connected to the material flow path 13. The mixing flow path 141 may extend horizontally forward from the location. The resultant flow path 14 may include a resultant discharge flow path 142 that connects an end of the mixing flow path 141 and the outlet 140 of the resultant flow path 14. The resultant discharge flow path 142 may extend upwardly from the front end of a mixing flow path 141.

Since the cell flow path 12 meets the material transfer flow path 132 and the mixing flow path 141 in a non-parallel state, the flow of the cell solution (SC) is bent at the meeting point. The cell solution (SC) may be bent at 90 degrees. Since the direction in which the cell solution (SC) flows changes at the point where the flow paths meet, the cells (C0) settle downward by the centrifugal acceleration to easily meet the material (M).

An electric field forming unit 20 can form an electric field along the solution flowing in each flow path (12, 13, 14) using each electrode (22, 23, 24). The electric field forming unit 20 can form an electric field in the material flow path 13 and the resultant flow path 14 using the material electrode 23 and the resultant electrode 24. The electric field forming unit 20 can form an electric field in the cell flow path 12 and the resultant flow path 14 using the cell electrode 22 and the resultant electrode 24. The electric field formed in a direction starting from the path 12 or the material flow path 13 and going out through the resultant flow path 14 is indicated by a dotted line in the drawing. It is possible to form a flow direction in the flow paths (12, 13, 14) of each solution (SC, SM) by forming an electric field, and it is possible to allow the transformation by electroporation to occur in the mixing flow path 141 to be described later.

In the mixing flow path 141, the transformant (M) of the material solution (SM) can enter the inside of cells (C0) of the cell solution (SC). Since the phospholipid bilayer of the cells (C0) is locally opened by the electric field applied to the mixing flow path 141, the transformant (M) can enter the inside of the cells (C0).

The thickness (T11+T21) of the mixing flow path 141 may be smaller than the thickness (T10) of the cell flow path 12. In particular, the thickness of the flow paths (12, 13, 14) refers to the degree to which the boundary of each of the flow paths (12, 13, 14) is spread in a direction orthogonal to the direction, in which the solutions (SC, SM) in the flow paths (12, 13, 14) flow, in the cross-section of each flow path (12, 13, 14) cut in a plane orthogonal to the left and right directions as shown in FIG. 4. When each of the flow paths (12, 13, 14) is formed in a pipe shape extending from a cylinder, the thickness of the flow paths (12, 13, 14) may be the inner diameter of the flow paths (12, 13, 14). The thickness (T11+T21) of the mixing flow path 141 may be smaller than the thickness (T20) of the material flow path 13. The thickness of the mixing flow path 141 may be 6 $\mu$m or more and 400 $\mu$m or less.

Since the thickness (T11+T21) of the mixing flow path 141 is smaller than the thickness (T10) of the cell flow path 12 converging to the resultant flow path 14 or the thickness (T20) of the material flow path 13, the thickness (T11) of the cell solution (SC) in the mixing flow path 141 may be smaller than the thickness of the cell flow path 12, which is the thickness occupied by the cell solution (SC). Similarly, the thickness (T21), which is a thickness of the material solution (SM) in the mixing flow path 141, may be smaller than the thickness (T20), which is a thickness occupied by the material solution (SM) in the material flow path 13. In particular, when the thickness (T11) of the cell solution (SC) in the mixing flow path 141 is smaller than the diameter of the cells (C0), part of the cells (C0) may be exposed to the material solution (SM) and placed in a state where they can meet the transformant (M). As an electric field is formed in the mixing flow path 141, the transformant (M) may enter the cells (C0) to form transformed cells (C1). Since part of the cells (C0) is exposed to the material solution (SM), transformation can occur even if the material solution (SM) and the cell solution (SC) are not mixed with each other.

To enable the above flow, the value obtained by multiplying (the thickness of the mixing flow path 141) and (the value obtained by dividing the cell flow path 12 by the sum of the thickness of the cell flow path 12 and the thickness of the material flow path 13) may be smaller than the diameter of the cells (C0) flowing in the cell flow path 12.

The thickness (T11) of the cell solution (SC) flowing in the mixing flow path 141 may be determined by controlling the flow rate at which each solution is injected. The processor 201 can control the pump unit 202 so that the thickness (T11) of the cell solution (SC), which is a solution flowing into the mixing flow path 141 through the cell flow path 12, in the mixing flow path 141 to be smaller than the diameter of the cells contained in the cell solution (SC). Under the situations with other conditions being the same, when the pressure for supply of the cell solution (SC) or a flow rate by the pump unit 202 increases, the thickness (T11) of the cell solution (S)=C) in the mixing flow path 141 can increase.

In order to allow the cells (C0) to be easily exposed to the material solution (SM) in the mixing flow path 141, those described and the cell flow path 12 may meet one other in a state located above the material flow path 13. This is because since the cell solution (SC) will be located above the material solution (SM) in the mixing flow path 141 by such flow path arrangement, the cells (C0) carried by the cell solution (SC) will move toward the material solution (SM) on a lower side thereof under the influence of gravity and be well exposed to the material solution (SM).

Transformation can occur and transformed cells (C1) can be obtained simply by injection into the body unit 10 and applying an electric field, without performing separate pretreatment of the cells or the transformant (M) due to the structure of the body unit 10 described above. Therefore, a pretreatment process such as cell washing can be omitted, and the losses in cells and material during the process can be significantly reduced. In addition, since it is not necessary to use a separate special buffer, it is possible to constitute an economical process. In addition, since no pretreatment is required, it is possible to constitute the process merely by enabling the injection of the solution into a closed and controlled environment, and thus it is advantageous from various regulations in that it can maintain a sanitary working environment. Simply injecting a culture medium containing cells and a material solution and applying an electric field are all necessary steps for transformation; therefore, it is easy to automate the process.

**Second embodiment**

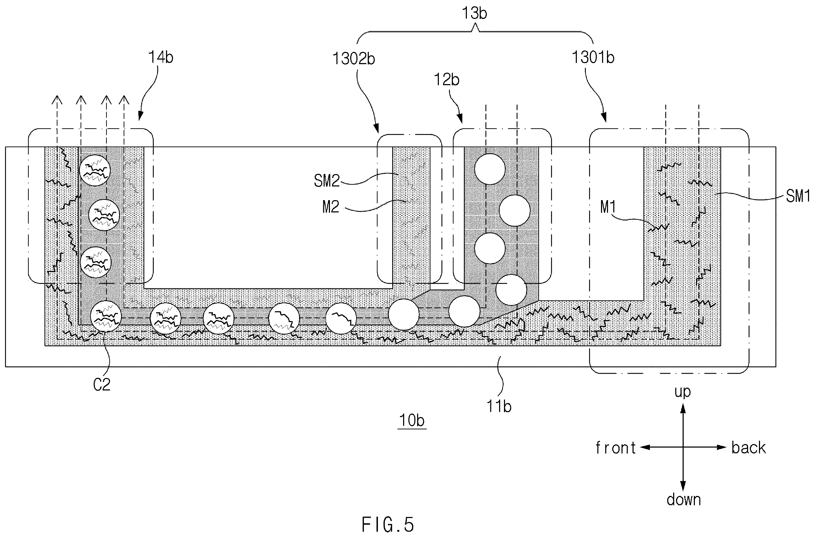

FIG. 5 is a conceptual diagram of a body unit 10b of a cell transformation cartridge according to the second embodiment of the present invention.

Since the cell transformation cartridge according to the second embodiment of the present invention is the same as the cell transformation cartridge 1 according to the first embodiment, except that it has a second material flow path 1302b, merely the parts with differences further described, and the description of the cell transformation cartridge 1 according to the first embodiment may be applied as-is for the remaining components. In the electric field forming unit, guide, O-ring unit cell transformation cartridge, etc. according to the second embodiment, the part for a second material flow path 1302b is formed.

The material flow path 13b formed in the body unit body 11b includes a first material flow path 1301b, through which a first material solution SM1 including a first transformant (M1) flows, and a second material flow path 1302b, through which a second material solution SM2 including a second transformant (M2) flows. The first transformant (M1) and the second transformant (M2) may be different from each other. The first material flow path 1301b may be the same as the material flow path 13 according to the first embodiment. Therefore, the first material flow path 1301b is considered to be the same as the material flow path 13 of the first embodiment, and a further explanation on the second material flow path 1302b will be provided herein below.

The first material flow path 1301b may meet the cell flow path 12b at one side of the cell flow path 12b, and the second material flow path 1302b may meet the cell flow path 12 at the other side of the cell flow path 12b. The first material flow path 1301b may meet the cell flow path 12b at a lower side of the cell flow path 12b, and the second material flow path 1302b may meet the cell flow path 12b at an upper side of the cell flow path 12b. Therefore, by the same action as described in the first embodiment in the resultant flow path 14, the first a material solution (SM1) may flow to an upper side of the cell solution (SC), and a second a material solution (SM2) may flow to a lower side of the cell solution (SC), and the cells may be exposed to the first material solution (SM1) and the second a material solution (SM2). Cells exposed to each a material solution (SM) may be opened by an electric field, and thus the first transformant (M1) and the second transformant (M2) may enter the cell and thereby transformation may occur.

Therefore, according to the second embodiment, the transformed cells (C2) can be formed by simultaneously injecting a plurality of different types of transformants (M1, M2) into cells using one cell transformation cartridge.

## Third embodiment

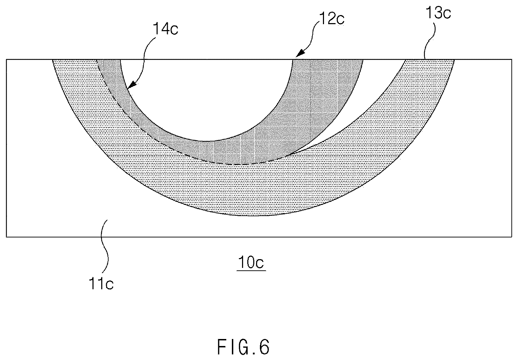

FIG. 6 is a conceptual diagram of a body unit 10c of a cell transformation cartridge according to the third embodiment of the present invention.

Since the cell transformation cartridge according to the third embodiment of the present invention is the same as the cell transformation cartridge 1 according to the first embodiment, except for the shape of the flow paths (12c, 13c, 14c) formed in the body unit 10c, merely the parts with differences will be further described, and the description of the cell transformation cartridge 1 according to the first embodiment may be applied as-is for the remaining components.

The appearance of the body unit body 11c according to the third embodiment may be the same as that of the body unit body 11 according to the first embodiment. The material flow path 13c according to the third embodiment may have a shape of an arc that is convex downward. The material flow path 13c and the resultant flow path 14c may have a shape of a continuous arc that is convex downward. That is, the material flow path 13c and the resultant flow path 14c may be connected by forming a continuous arc that is convex downward. Although the cell flow path 12c is also shown to have a curved profile, such as having an arc shape, the cell flow path 12c may have a shape extending downward. When the cell flow path 12c has an arc shape, the radius of curvature of the cell flow path 12c may be smaller than that of the material flow path 13c.

Since the material flow path 13c has an arc shape, centrifugal acceleration is applied to the transformant that is injected into the material flow path 13c, and thus it may be easier for the transformant to meet the cells.

The arc-shaped flow path as in the third embodiment may also be applied to the first material flow path 1301b and the second material flow path 1302b of Second embodiment.

## Fourth embodiment

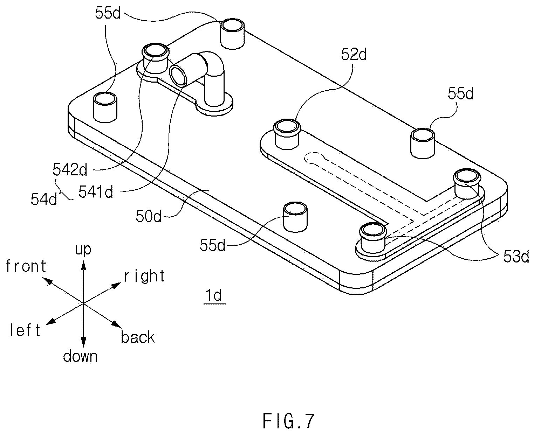

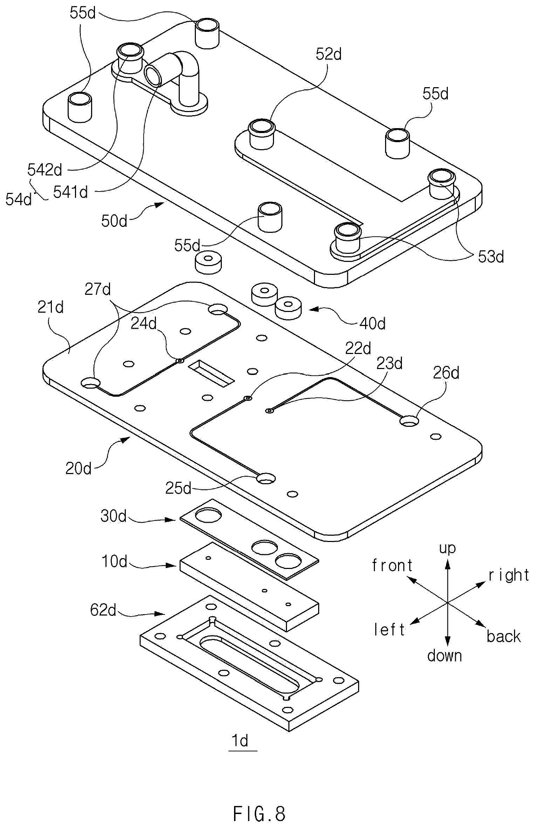

FIG. 7 is a perspective view of a cell transformation cartridge 1d according to the fourth embodiment of the present invention. FIG. 8 is an exploded perspective view of a cell transformation cartridge 1d according to the fourth embodiment of the present invention.

The cell transformation cartridge 1d according to the fourth embodiment of the present invention has components the same as the body unit 10, O-ring unit 40, and the sealing sheet 30 of the cell transformation cartridge 1 according to the first embodiment. Since there are some conformational differences in the remaining components, merely the parts with differences will be further described, and the description of the cell transformation cartridge 1 according to the first embodiment may be applied as-is for the remaining components.

The guide unit 50d of the cell transformation cartridge 1d according to the fourth embodiment of the present invention may receive and mix a plurality of solutions and deliver it to a material flow path of the body unit 10d. To this end, the guide part 50d may include a plurality of material guides 53d. Since each of the material guides 53d of the cell transformation cartridge 1d according to the fourth embodiment is injected with a mutually-different material solution, and the mutually-different material solution is integrated into one flow path and delivered while being delivered to the inlet of the material flow path through the guide part 50d, and thus these mutually-different material solutions may be mixed with one other during the process being flowed.

The guide unit 50d may discharge the resultant solution through a plurality of guides. Therefore, the resultant guide 54d may include a main resultant guide 541d and an auxiliary resultant guide 542d. The main resultant guide 541d may have a pipe shape that is bent to the left after extending upward. The auxiliary resultant guide 542d may have a pipe shape extending upward. The resultant solution discharged from the resultant flow path may be separated from the guide part 50d into the main resultant guide 541d and the auxiliary resultant guide 542d and discharged, respectively.

The guide unit 50d may include a power application guide 55d. The power application guide 55d may be formed in a plural number. The guide unit 50d can allow each of the terminals (25d, 26d, 27d) of an electric field forming unit 20d located on a lower side of the guide body 51d to be exposed to the outside of the guide unit (50d) and allow the connector to be connected to each of the terminals (25d, 26d, 27d) to be stably in contact with each of the terminals (25d, 26d, 27d) and fixed. The power application guide 55d may also be formed in a pipe shape extending upward from the guide body 51d.

The electric field forming unit 20d may include a material terminal 26d electrically connected to the material electrode 23d, a resultant terminal 27d electrically connected to the resultant electrode 24d; and a cell terminal 25d electrically connected to the cell electrode 22d. Each of the terminals (25d, 26d, 27d) may be disposed at a location corresponding to the power application guide 55d on the electric field forming body 21d. The electric field forming unit 20d may be coupled through the guide portion (50d) and a fastener.

The body mount 62d may include a hole in the center into which the body unit 10d is inserted, so that the body unit 10d may be aligned with other components in an appropriate location. However, a step is formed in the hole of the body mount 62d, so that the body unit 10d and the sealing sheet 30d are not separated downward while the body unit 10d and the sealing sheet 30d are inserted. The body mount 62d may be coupled to the electric field forming unit 20d through fasteners.

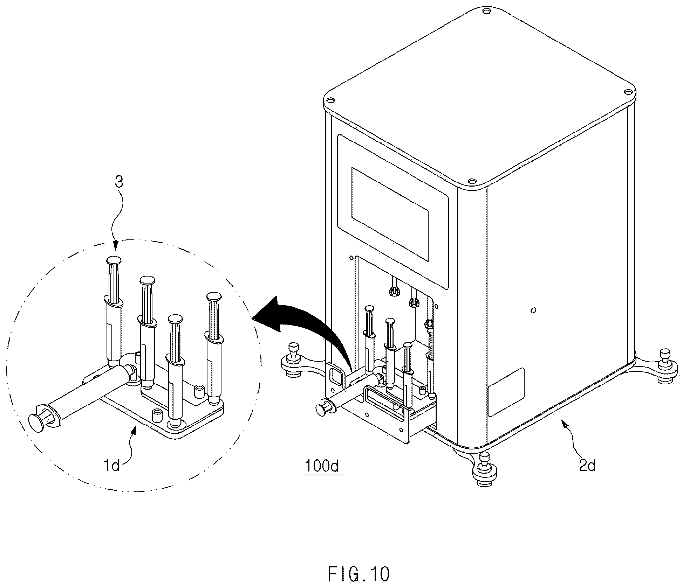

FIG. 9 is a view showing a cell transformation system 100 including a cell transformation cartridge 1 and a cell transformation device 2 according to the first embodiment of the present invention. FIG. 10 is a view showing a cell transformation system 100d including a cell transformation cartridge 1d and a cell transformation device 2d according to the fourth embodiment of the present invention.

Referring to the drawings, the cell transformation systems (100, 100d) according to the first embodiment and the fourth embodiment of the present invention include a cell transformation cartridge (1, 1d) and a cell transformation device (2, 2d). The cell transformation system (100, 100d) may include a syringe 3. Since the difference between the first embodiment and the fourth embodiment lies only in the number of syringe 3, the number of pumps, the number of guides, etc., the cell transformation method for the cells of the present invention will be described with reference to FIG. 9 as a representative.

Transformation may occur by injecting each solution into a cell transformation cartridge 1 by a cell transformation device 2. A cell transformation cartridge, in which a cell flow path 12, a material flow path 13, and a resultant flow path 14 are formed, is prepared. That is, the cell transformation method may include preparing the cell transformation cartridge 1. In the guide unit 50 of such a cell transformation cartridge 1, a syringe 3 including each cell solution and a material solution can be inserted. The cell transformation cartridge 1 into which the syringe 3 is inserted can be introduced into the inside of the cell transformation device 2.

The cell transformation device 2 according to the first embodiment of the present invention includes a pump unit 202 and a processor 201. The cell transformation device 2 may include an input unit 204 and may include a power application unit 203.

The pump unit 202 is provided to pressurized supply of a cell solution SC and a material solution SM to a cell flow path 12 and a material flow path 13, respectively. Therefore, the pump unit 202 may include a plurality of pumps for pressurized supply of each solution. The pumps of the pump unit may deliver a solution from a storage tank, in which each solution is accommodated, to each flow path of the body unit 10 to be described later through respective pipes.

Each pump may be a syringe pump capable of contacting the syringe 3 and pushing the piston of the syringe 3 at a desired speed. Therefore, once the cell transformation cartridge 1 in which the syringe 3 is inserted enters the inside of the cell transformation device 2, the fluid can be delivered by pressurization in such a manner that the pump approaches and contacts the piston of the syringe 3 and pushes the syringe 3 as the operation starts. However, the pump is not limited to the syringe pump, and the pump may be constituted in such a way that the pipe is directly coupled to each flow path of the cartridge and the fluid is delivered by pressurization.

The pump unit 202 can allow a pressing member for pushing the piston to be brought into contact with the piston, when the cell transformation cartridge 1 enters the inside of the cell transformation device 2. In particular, the pump unit 202 can measure the distance traveled by the pressing member so that the processor 201 can calculate the volume of the solution stored in the syringe 3.

The processor 201 may be electrically connected to the pump unit 202 to control at least one of the pressure or flow rate at which the pump unit 202 pressurizes and supplies each solution. The processor 201 is a component including an element capable of performing a logical operation for performing a control command, and may include a central processing unit (CPU), etc. The processor 201 is connected to various components of the cell transformation device 2 according to an embodiment of the present invention, and thus can transmit the signal according to the control command to each component, and receive the information obtained by being connected to various sensors or acquisition units in the form of signals. Since the processor 201 can be electrically connected to each of the components, it may be connected with a wire or may communicate with each other by having a communication module capable of wireless communication.

The cell transformation device 2 may further include a storage medium, and thus control commands performed by the processor may be stored in the storage medium and utilized. The storage medium may be a device such as a hard disk drive (HDD), a solid state drive (SSD), a server, a volatile medium, a nonvolatile medium, etc., but the type is not limited thereto. In addition thereto, data required for the processor 201 to perform an operation may be further stored in the storage medium.

The cell transformation device 2 can receive necessary information through the input unit 204. The input unit 204 can receive information including means such as a button, a switch, and a touch screen, and information can also be input by scanning codes, including barcodes, QR codes, and RFID scanners. Information regarding the cell transformation cartridge 1 entering the cell transformation device 2 can be input through the input unit 204. The information input to the input unit 204 is transmitted to the processor 201.

The processor 201 can determine the flow rate of each solution being delivered to the cell transformation cartridge 1 by the pump unit using the input information and the information preset and stored therein. The processor 201 can determine the flow rate of each solution as a value that allows the cells (C0) and the material (M) to meet to cause transformation in the mixing flow path 141. Once the processor 201 knows which material solution (SM) and which cell solution (SC) will be used, it can retrieve the optimal flow ratio corresponding to the material solution (SM) and the cell solution (SC) from the stored data. The processor 201 can also retrieve the optimal exposure time for the transformation process from the stored data. The processor 201 can determine the flow rate of the cell solution (SC) and the flow rate of a material solution (SM) by combining the retrieved flow rate and the exposure time with the input information of the cell transformation cartridge 1 (a flow path length of the cartridge, a shape of the flow path shape, etc.), and it is possible to operate the pump unit 202 according to the determined flow rate to thereby push the syringe 3. In particular, the flow ratio determines to what extent of the thickness of the total thickness of a mixing flow path 141 will be occupied by the material solution (SM) and the cell solution (SC) within the mixing flow path 141. The optimal exposure time means the time sufficient for transformation to occur while the cells (C0) and the material (M) pass through the mixing flow path 141.

The flow ratio may be a value that allows transformation to occur when the cells (C0) and the material (M) meet in the mixing flow path 141. The flow ratio may be a value which allows the thickness (T11), that is possessed within the mixing flow path 141 by the solution being flowed into the mixing flow path through the cell flow path, to be equal to or less than three times the diameter of the cells (C0), and preferably, may be a value that allows the thickness (T11) to be smaller than the diameter of the cell (C0).

The cell transformation method may include operating the pump unit 202 according to the determined ratio of flow rates and flow rate values, injecting the cell solution (SC) into the cell flow path 12, and injecting the material solution (SM) into the material flow path 13.

The power application unit 203 is electrically connected to the processor 201, and may be connected to the terminals (26, 27) to apply power to the electrodes (22, 23, 24) included in the electric field forming unit 20 of the cell transformation cartridge 1. Once the cell transformation cartridge 1 enters the inside of the cell transformation device 2, a connection arm of the power application unit 203 approaches and contacts with the terminals (26, 27) to thereby allow an electrical connection. When injecting the cell solution (SC) into the cell flow path (12) using the pump unit (202) and injecting the material solution (SM) into the material flow path 13, the power application unit 203 may apply power to the electric field forming unit 20 so that an electric field can be formed. As the solution is supplied and an electric field is formed, and transformation occurs in the mixing flow path 141, and a solution containing the transformed cells (C1) may be discharged through the outlet 140 of the resultant flow path 14.

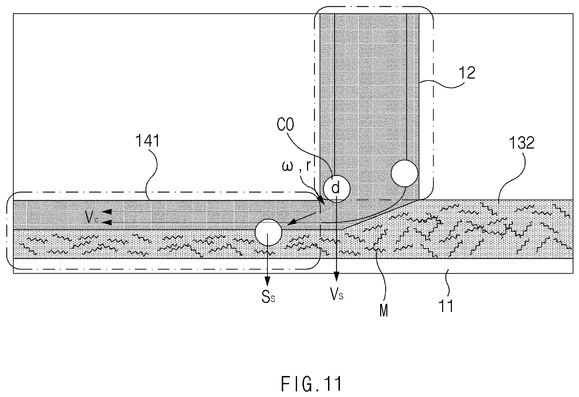

FIG. 11 is a view showing the settlement of cells occurring in a cell transformation cartridge 1 according to the first embodiment of the present invention.

Using FIG. 11, the flow rate of a solution that can be provided by a cell transformation device 2 when the cell transformation cartridge 1 according to the first embodiment of the present invention is provided to the cell transformation device 2 is described. When the cell flow path 12 and the material transfer flow path 132 meet and lead to the mixing flow path 141, the flow of the cell solution (SC) flowing in a cell flow path (12) may be bent at a right angle, and the flow of the cell solution (SC) may be approximated as if it had a circular motion at the corner. In particular, SS, which is the distance at which the cells (C0) settle downward by the centrifugal force of the above-mentioned circular motion in the mixing flow path 141, may be expressed as follows using the equation related to centrifugation. SS may be 1 μm or more and 20 μm or less.

[Equation 1]

$$S_s = V_s * t_s \sim \frac{d^2 \Delta\rho \omega^2 r}{18\mu} * \frac{\pi}{2\omega} = \frac{\pi d^2 \Delta\rho}{36\mu} V_c$$

In Equation 1 above, $V_S$ is the velocity of the cells (C0) facing downward as shown, $t_S$ is the time required to pass through the corner when the corner is approximated as a quadrant, r is the radius of the approximate quadrant, d is the diameter of the cells (C0), μ is the viscosity of the cell solution (SC), $\Delta\rho$ is the density difference between the cells (C0) and the cell solution (SC), ω is the angular velocity of the cells (C0), and $V_c$ is the horizontal velocity possessed by the cells (C0) in the mixing flow path 141.

The cell transformation device 2 can provide the cell solution (SC) and the material solution (SM) to the cell transformation cartridge 1 at a ratio of flow rates that makes the thickness of the cell solution (SC) smaller within the mixing flow path 141 compared to the above-mentioned $S_S$. Each solution is provided at such a ratio of flow rates so that the cells (C0) can meet the material (M) in the mixing flow path 141 and thereby allow transformation to occur.

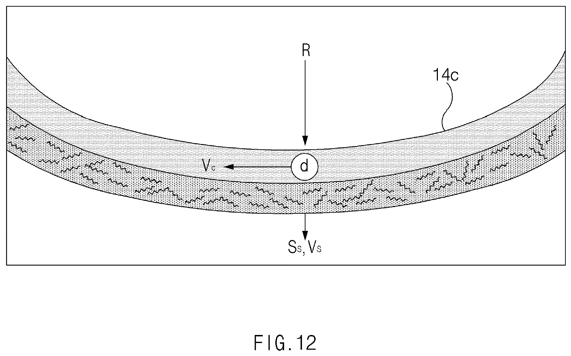

FIG. 12 is a view showing the settlement of cells occurring in a cell transformation cartridge according to the third embodiment of the present invention.

The flow rate of the solution that the cell transformation device 2 can provide when the cell transformation cartridge according to the third embodiment of the present invention is provided to the cell transformation device 2 is described. Since a mixing flow path that is a part of a resultant flow path 14c may have a circular arc shape as shown, the flow of the cell solution may be approximated as if it had a circular motion. In particular, $S_S$, which is the distance at which cells settle downward by the centrifugal force of the above-mentioned circular motion in the mixing flow path, can be expressed as follows. $S_S$ may be 2 μm or more and 30 μm or less.

[Equation 2]

$$S_s = V_s * t_s = \frac{d^2 \Delta\rho}{18\mu} \frac{V_c^2}{R} * \frac{L_E}{V_c} = \frac{\pi d^2 \Delta\rho}{36\mu} \frac{L_E}{R} V_c$$

In Equation 2 above, LE is the length of a mixing flow path, and R is the radius of curvature of a mixing flow path.

The cell transformation device 2 can provide a cell solution and a material solution to the cell transformation cartridge at a ratio of flow rates that makes the thickness of the cell solution smaller in the mixing flow path than the $S_S$ described above. Each solution is provided at such a ratio of flow rates, so that the cells can be transformed by meeting materials in the mixing flow path.

**Experimental Example 1**

First, the transformation efficiency of mRNA using the cell transformation cartridge of the present invention was confirmed.

To this end, using the cell transformation cartridge of the present invention described above, 1 mL of a cell solution containing NK-92 cells (ATCC CAT# CRL-2407) at a concentration of $4 \times 10^7$ cells/mL in a cell culture medium was added to a cell flow path, and a material solution containing eGFP mRNA (RiboPro, CAT# RB-079) at a concentration of 200 $\mu$g/mL was added to a material flow path, respectively. Then, while applying an electric field of 1,200 V between the inlet of the material flow path and the resultant discharge flow path, the cell solution and the material solution were allowed to pass through the mixing flow path (depth 30 $\mu$m, width 3 mm, length 24 mm) applied with an electric field at a ratio of flow rates of 1:2, thereby a transformant in which the eGFP mRNA was introduced into NK-92 cells was obtained, which was used as an experimental group.

Meanwhile, using Neon™ Transfection System (ThermoFisher), a material solution containing the same eGFP mRNA used in the preparation of the transformant of the experimental group was mixed with a cell solution containing NK-92 cells in the same cell culture medium used in the preparation of the transformant of the experimental group, instead of R-buffer (*i.e.,* a buffer solution for Neon™ Transfection System). For the rest, transformants were obtained according to the manufacturer's instructions, and this was used as a positive control. Additionally, as a negative control, a cell solution containing NK-92 cells into which mRNA was not introduced was used.

For each of the experimental group and positive and negative control groups obtained as above, flow cytometry was performed; the expression efficiency (eTX) was analyzed by classifying cells with stronger fluorescence as successful expression cells based on the signal value of the top 1% of the negative control group, and the cell viability was measured by Texas Red fluorescence staining technique.

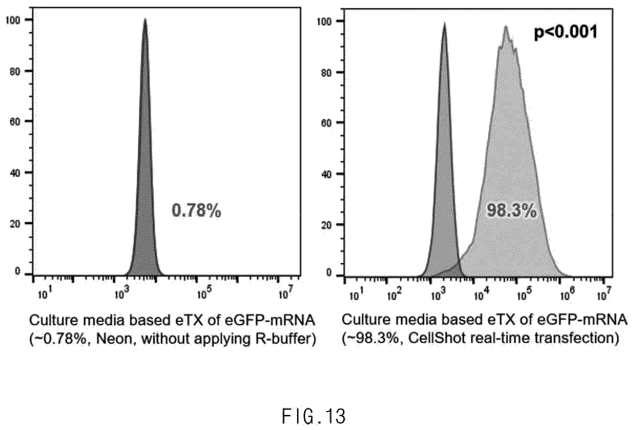

As a result, it was confirmed that the negative control group and the experimental group prepared using the cell transformation cartridge of the present invention showed a high cell viability of about 95%, and the positive control prepared with the Neon™ Transfection System using cells on the culture medium as-is without pretreatment also showed a high cell viability of about 90%. However, as shown in FIG. 13, it was confirmed that the expression efficiency (eTX) was 0.78% in the positive control group, and there were almost no transformants expressing GFP fluorescence (left graph of FIG. 13), whereas in the experimental group prepared using the cell transformation cartridge of the present invention, the expression efficiency (eTX) was 98.3% and almost all cells were transformants expressing GFP fluorescence (right graph of FIG. 13).

Since mRNA is easily destroyed in cell culture medium containing various components necessary for cell growth, in conventional mixed delivery technologies such as the Neon™ Transfection System, the cells to be transformed should be washed, transferred to a dedicated buffer (R-buffer for Neon™ Transfection System), and mixed with mRNA in that state. Nevertheless, it appears that in the positive control as above, since NK-92 cells were used as they were contained in the cell culture medium without using R-buffer (*i.e.,* a buffer solution for Neon™ Transfection System), in a mixture of eGFP mRNA and NK-92 cells, all of the eGFP mRNA was destroyed, and thus the transformant itself in which the eGFP mRNA was introduced into the NK-92 cells could not be prepared. From the above results, it can be understood that in the case of existing mixed delivery technologies such as Neon™ Transfection System, it is virtually impossible to directly deliver mRNA to cells on the culture medium without pretreatment such as washing the cells or using a dedicated buffer.

In contrast, when using a cell transformation cartridge of the present invention described above, NK-92 cells are used as-is contained in the cell culture medium; however, since the cell solution and the material solution containing eGFP mRNA are supplied through separate flow paths, the eGFP mRNA can be delivered to NK-92 cells without being destroyed by components in the cell culture medium, as a result, the eGFP mRNA can be introduced into almost all NK-92 cells, and thus transformants can be prepared with high efficiency. From the above results, it can be seen that it is possible to directly deliver mRNA to cells on the culture medium using a cell transformation cartridge of the present invention. Accordingly, it was clearly confirmed through the examples of the present invention that when using the cell transformation cartridge of the present invention, it does not require any pretreatment (e.g., washing the cells or using a dedicated buffer); therefore, cell loss or cell damage is not caused during the cell washing process, and there is no need to use expensive consumables such as dedicated buffers, and the transformation process itself can also be significantly simplified.

In particular, it can be seen that compared with the existing mixed delivery technology in which transformation is performed after the transfer to a dedicated buffer, after undergoing a cell washing process that causes cell loss or cell damage, the transformation performed using the cell transformation cartridge of the present invention has significant advantages in that not only in expression efficiency and cell viability (*i.e.,* two important factors in intracellular mass transfer), but also in terms of a process automation rate, a process sealing rate, and manufacturing yield (*i.e.,* the three most important factors in the cell therapy manufacturing process) .

**Experimental Example 2**

Next, in the process of performing transformation by separately supplying a cell solution and a material solution using the cell transformation cartridge of the present invention, the effect of the ratio of flow rates between the two solutions in the mixing flow path on the delivery efficiency of materials such as mRNA was confirmed.

The flow of a fluid in the mixing channel is a laminar flow that has a parabolic velocity profile in the form of a quadratic function depending on the depth, in which the velocity becomes maximum at the center of the flow path, and the velocity becomes 0 at the top and bottom surfaces. Therefore, depending on the ratio of flow rates between the cell solution and the material solution, there may be a change in the velocity profile of each flow thereby affecting the transfer efficiency of the material. In addition, in terms of geometrical aspect, since the height of the flow becomes smaller as the flow rate decreases due to the fixed height of the mixed flow path, in the case of cell flow, the thickness of the fluid in the cell flow can be adjusted by controlling the flow rate, so that the thickness can be adjusted according to the cell size. The basic trend is that the smaller the thickness of the fluid in the cell flow, the more efficient it is to interact with the material flow in the cells, thereby resulting in higher efficiency.

To measure this, the delivery and expression efficiency of eGFP mRNA were measured by varying the ratio of flow rates (a flow rate of the material/a flow rate of cells) to 0.5, 1, and 2. Specifically, using the cell transformation cartridge of the present invention described above, 1 mL of a cell solution containing NK-92 cells (ATCC CAT# CRL-2407) at a concentration of $4 \times 10^7$ cells/mL in a cell culture medium was injected to the cell flow path, and a material solution containing eGFP mRNA (RiboPro, CAT# RB-079) at a concentration of 200 μg/mL were injected into the material flow path, respectively, were allowed to pass through a mixing flow path (depth 30 μm, width 3 mm, length 24 mm) applied with an electric field at a ratio of flow rates (a flow rate of the material/a flow rate of cells) of 2, 1, and 0.5, while applying an electric field of 1,200 V between the inlet of the material flow path and the resultant discharge flow path; as a result, transformants in which the eGFP mRNA was introduced into NK-92 cells were obtained, and these were designated as transformants #1, #2 and #3, respectively.

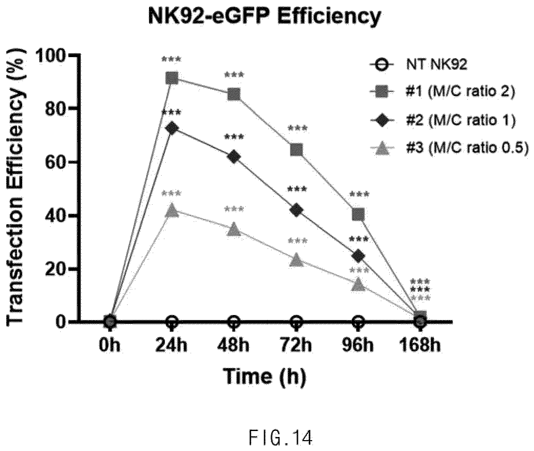

For each of the transformants #1, #2 and #3 obtained as described above, flow cytometry was performed in the same manner as in Example 1 to analyze delivery and expression efficiency. As a result, as shown in FIG. 14, the smaller the flow rate of the cell flow, the higher the transfer efficiency. In the case of NK-92 cells with an average size of about 15 μm, it is analyzed that the thickness of the cell flow was about 10 μm, 15 μm, and 20 μm at each mixing ratio, whereas in the case of having a thickness of 10 μm, which is smaller than the average cell diameter, almost all cells showed an expression rate of 90% or more, which has become the expression level. It could be seen that when the cell solution flow had a thickness similar to the diameter of the cell, a high expression rate of about 75% was maintained, whereas when the cell solution flow had a higher thickness than the cell diameter, the expression efficiency was reduced to a level of 40%.

From the above results, it can be confirmed that the process of material transfer in the mixing flow path of the cell transformation cartridge of the present invention can induce the material transfer by allowing the cells in the cell solution to move effectively toward the material solution and contact even if the mixing between the two fluids does not occur effectively. That is, for a fluid flow in the region of laminar flow, since there is no fluid mixing effect due to the inertial force occurring in the turbulent flow region, two or more fluid flows introduced separately cannot be effectively mixed within a short period of time, even under laminar flow conditions where the cell solution and the material solution cannot be directly mixed as in a cell transformation cartridge of the present invention, an effective intracellular material transfer and transformation can be made possible by setting the cells in the cell solution such that they can move directly toward the material solution and immediately contact with the material solution. In addition, it can be confirmed through this experimental example that almost all cells can be transformed at a cell fluid flow thickness less than the cell diameter, and it can also be seen that transformation occurs in some cells under the cell solution flow condition having a thickness higher than the cell thickness, and the proportion of transformed cells decreases as the thickness increases.

**Experimental Example 3**

Furthermore, it was confirmed whether a material could be delivered with high efficiency even to cells collected from the human body (primary cells) when the cell transformation cartridge of the present invention is used.

In the case of conventional mixed delivery technologies such as the Neon™ Transfection System, cell loss and damage were inevitably induced in the process of washing the cells to remove the cell culture medium, in the case of cells established as a cell line for use by culturing in the laboratory due to their indefinite proliferation potential, it is known that the cell viability is relatively good; however, in the case of primary cells collected from human blood or tissue, there were problems in that not only it is generally more difficult to proliferate through culture than in the above cell lines, but also more cell damage is induced in the process of cell washing, thus the yield is significantly reduced in the process of producing a transformant to be used as a cell therapeutic agent. However, as confirmed in Example 1 above, in the case of performing transformation using the cell transformation cartridge of the present invention, since materials can be delivered directly to the cells on the cell culture medium without pretreatment such as cell washing or the use of a dedicated buffer, it is expected that materials can be delivered with high efficiency even to primary cells collected from the human body.

To confirm this, using the cell transformation cartridge of the present invention described above, 1 mL of a cell solution containing human Peripheral Blood Monoclonal Cells (hPBMC) (Lonza, CAT# CC-2702) at a concentration of $5 \times 10^7$ cells/mL in a cell culture medium was added to the cell flow path, and a material solution containing eGFP mRNA (RiboPro, CAT# RB-079) at a concentration of 200 $\mu$g/mL was added to the material flow path, respectively. Then, while applying an electric field of 1,800 V between the inlet of the material flow path and the resultant discharge flow path, the cell solution and the material solution were allowed to pass through the mixing flow path (depth 30 $\mu$m, width 3 mm, length 24 mm) applied with an electric field at a ratio of flow rates of 2:1, thereby a transformant in which the eGFP mRNA was introduced into hPBMC was obtained, which was used as an experimental group.

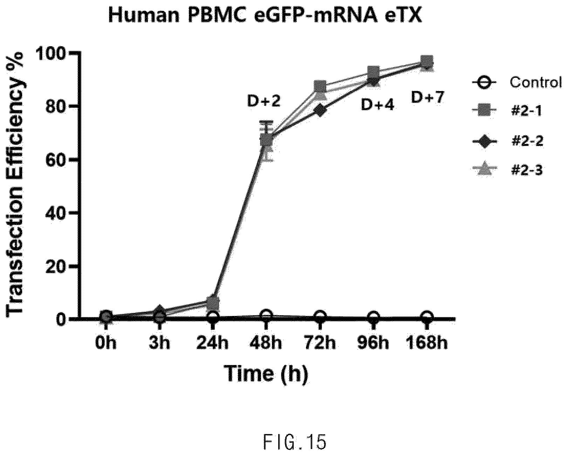

For the transformant obtained as described above, eGFP expression efficiency (eTX) and cell viability were measured in the same manner as in Example 1. As a result, as shown in FIG. 15, it was confirmed that compared to the negative control group without material transfer, the transformant, which is hPBMC into which eGFP mRNA was introduced using a cell transformation cartridge of the present invention, not only exhibited an equivalent level of cell viability, but also simultaneously maintained the GFP expression for more than a week while exhibiting such a high cell viability and the expression efficiency reached a level of up to 98% or more.

From the above results, it can be seen that in the case of performing transformation using the cell transformation cartridge of the present invention, materials can be delivered directly to the cells on the cell culture medium without pretreatment such as cell washing or the use of a dedicated buffer; therefore, intracellular material transfer can be effectively performed without causing cellular damage to a level that leads to apoptosis, and that due to these advantages, materials can be delivered with high efficiency even to the cells (primary cells) collected from the human body. In addition, in the case of the thus-prepared transformant of human harvested cells, the maximum expression efficiency can be exhibited at the time point where about a week has elapsed, it can be seen that when applied as a cell therapy, the efficacy can last for more than a week. This is an excellent effect that could not be achieved with existing mixed delivery technologies such as the NeonTM Transfection System, and it can clearly be seen that through this experimental example that transformation technology using a cell transformation cartridge of the present invention can be very useful in the process of using human cells in which the number of cells that can be used is extremely limited.

**Experimental Example 4**

Based on the excellent transformation efficiency for various cells such as cell lines and cells collected from humans confirmed in the above Examples, CAR-NK cells were prepared by introducing the mRNA of chimeric antigen receptors (CAR) into NK-92 cells (ATCC CAT# CRL-2407) using the cell transformation cartridge of the present invention.

Since NK cells are one of the most difficult cells to transform with various transformation technologies currently applicable to cell manipulation; therefore, there is a significant difficulty in preparing CAR-NK. Since the proportion of NK cells among blood cells is less than 5%, the absolute amount of NK cells that can be obtained from the human body is small; additionally, transformation efficiency of NK cells by viral infection is known to be less than 1%. Therefore, it is known that when a transformant is produced from human NK cells using existing mixed delivery technology such as the Neon™ Transfection System, the production yield is remarkably poor due to cell loss and cell damage that occurs during cell washing. However, as confirmed in the above examples, when the cell transformation cartridge of the present invention is used, it was confirmed that cell loss can be minimized and materials can be delivered with high viability and efficiency even in human-derived cells, and thus it is expected that CAR-NK therapeutics can also be produced in high yield by confirming the expression of CAR, whose anticancer therapeutic effects has been confirmed.

To confirm this, using the cell transformation cartridge of the present invention described above, 1 mL of a cell solution containing NK-92 cells (ATCC CAT# CRL-2407) at a concentration of $1.0 \times 10^7$ cells/mL in a cell culture medium was added to the cell flow path, and a material solution containing CD19 CAR mRNA (RiboPro, CAT# RB-073-3) at a concentration of 500 $\mu$g/mL was added to the material flow path, respectively. Then, while applying an electric field of 1,200 V between the inlet of the material flow path and the resultant discharge flow path, the cell solution and the material solution were allowed to pass through the mixing flow path (depth 30 $\mu$m, width 3 mm, length 24 mm) applied with an electric field at a ratio of flow rates of 2:1, thereby a transformant in which the CD19 CAR mRNA was introduced into NK-92 cells was obtained, and this was designated as NKL-CD19-500.

For the transformant obtained as described above, the expression efficiency (eTX) of the CD19 CAR was measured using an anti-FMC63-PE antibody, and the cytotoxicity of Nalm6 cancer cells was measured while culturing the transformant and the CD19-overexpressing cancer cell line Nalm6 (ATCC CAT# CRL-3273) after mixing them at a 2: 1 (CAR-NK cells: Nalm6 cells) ratio.

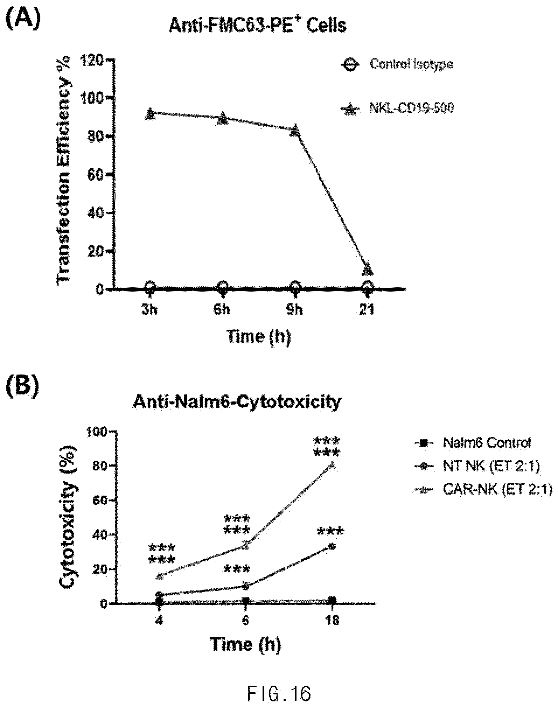

As a result, as shown in FIG. 16, it was confirmed that CD19 CAR was expressed in more than 90% of cells (FIG. 16(A)), and it was confirmed that more than 80% of Nalm6 cancer cells were killed within one day (FIG. 16 (B)).

From the above results, it can be seen that since materials can be delivered directly to the cells on the cell culture medium without pretreatment (e.g., cell washing or the use of a dedicated buffer) when performing transformation using the cell transformation cartridge of the present invention, efficient production of transformants using human NK cells, which was virtually impossible with existing mixed delivery technologies (e.g., Neon™ Transfection System) is possible. This is an excellent effect that could not have been achieved with existing mixed delivery technologies (e.g., Neon™ Transfection System), and the great potential for the development and production of CAR-NK anticancer immune cell therapeutics, which are expected to give a breakthrough in the treatment of solid cancer, was confirmed through this experimental example, and it can be clearly seen that transformation technology using a cell transformation cartridge of the present invention can be very useful in the field of human-collected NK cell-based anticancer immune cell therapy with high production performance that has not been reported in existing NK cell transformation technologies.

In the above, merely because all components constituting the embodiments of the present invention are described as being combined or combined to operate as one, the present invention is not necessarily limited to these embodiments. That is, as long as being within the scope of the purpose of the present invention, all the components may operate by selectively combining one or more. In addition, terms such as "comprise", "constitute", and "have" described above mean that the corresponding components may be inherent unless otherwise stated, and thus, it should not be construed as excluding other components, but may further include other components. All terms, including technical or scientific terms, unless otherwise defined, have the same meaning as commonly understood by those of ordinary skill in the art to which the present invention pertains. Commonly used terms, such as those defined in the dictionary, should be interpreted as being consistent with the contextual meaning of the related art, and should be construed in an idealistic or overly formal sense unless explicitly defined in the present invention.

As the above description is merely illustrative of the technical idea of the present invention, those of ordinary skill in the art to which the present invention pertains will be able to make various modifications and variations without departing from the essential characteristics of the present invention. Therefore, the embodiments disclosed in the present invention are not intended to limit the technical idea of the present invention, but to explain, the scope of the technical idea of the present invention is not limited by these embodiments. The scope of protection of the present invention should be construed by the following claims, all technical ideas within an equivalent scope should be construed as being included in the scope of the present invention.

[Reference Numeral]

1, 1d : cell transformation cartridge
2, 2d : cell transformation device
3 : syringe
10, 10b, 10c, 10d : body unit
11, 11b, 11c : body of body unit
12, 12b, 12c : cell flow path
13, 13b, 13c : material flow path
14, 14b, 14c : resultant flow path
20, 20d : electric field forming unit
21, 21d : electric field forming body
22, 22d : cell electrode
23, 23d : material electrode
24, 24d : resultant electrode
25d : cell terminal
26, 26d : material terminal
27, 27d : resultant terminal
30, 30d : sealing sheet
40, 40d : O-ring unit
42 : cell O-ring
43 : material O-ring
44 : resultant O-ring
50, 50d : guide unit
51, 51d : guide body
52, 52d : cell guide
53, 53d : material guide
54, 54d : resultant guide
55d : power application guide
61 : upper mount
62, 62d : body mount
63 : lower mount
100, 100d : cell transformation system
120 : inlet of cell flow path
121 : cell introduction flow path
130 : inlet of material flow path
131 : material introduction flow path
132 : material transfer flow path
140 : outlet of resultant flow path
141 : mixing flow path
142 : resultant discharge flow path

201 : processor
202 : pump unit
203 : power application unit
204 : input unit
541d : main resultant guide
542d : auxiliary resultant guide
1301b : first material flow path
1302b : second material flow path
CO : cells before transformation
C1, C2 : transformed cells
M : transformant
M1 : first transformant
M2 : second transformant
SC : cell solution
SM : material solution
SM1 : first material solution
SM2 : second material solution

**Claims**

1. A cell transformation cartridge, comprising:

   a body unit, in which a cell flow path, a material flow path, and a resultant flow path that meet one another are formed; and
   an electric field forming unit, which comprises an electrode being connected to the body unit to create an electric field in a resultant flow path,
   wherein the cell flow path and the material flow path are formed in a shape that converges to the resultant flow path,
   wherein the resultant flow path comprises a mixing flow path extending from a location being connected to the cell flow path and the material flow path, and
   wherein the thickness of the mixing flow path is smaller than that of the cell flow path.

2. The cell transformation cartridge of claim 1, wherein the cell flow path and the material flow path meet with each other in a state where the cell flow path is located above the material flow path.

3. The cell transformation cartridge of claim 1, wherein the cell flow path comprises a cell introduction flow path extending downward from an inlet thereof.

4. The cell transformation cartridge of claim 1, wherein the mixing flow path extends in one horizontal direction from a location being connected to the cell flow path and the material flow path.

5. The cell transformation cartridge of claim 1, wherein the material flow path has the shape of an arc convex downward.

6. The cell transformation cartridge of claim 5, wherein the material flow path and the resultant flow path have the shape of a continuous arc of a downward convex shape.

7. The cell transformation cartridge of claim 1, wherein the material flow path comprises a material introduction flow path extending downward from the inlet; and a material transfer flow path extending horizontally from a lower end of the material introduction flow path toward the resultant flow path.

8. The cell transformation cartridge of claim 1, wherein an inlet of the material flow path, an inlet of the cell flow path, and an outlet of the resultant flow path are arranged in the order of the inlet of the material flow path, the inlet of the cell flow path, and the outlet of the resultant flow path along the reference direction.

9. The cell transformation cartridge of claim 1, wherein the material flow path comprises:

   a first material flow path that meets the cell flow path on one side of the cell flow path; and
   a second material flow path that meets the cell flow path at the other side of the cell flow path.

10. The cell transformation cartridge of claim 9, wherein the first material flow path meets the cell flow path on a lower side of the cell flow path, and the second material flow path meets the cell flow path on an upper side of the cell flow path.

11. The cell transformation cartridge of claim 1, wherein the electric field forming unit is provided to form an electric field using a pulse-type power source.

12. The cell transformation cartridge of claim 1, wherein the resultant flow path further comprises a resultant discharge flow path that connects an end of the mixing flow path and the outlet of the resultant flow path.

13. The cell transformation cartridge of claim 1, wherein the thickness of the mixing flow path is 6 $\mu$m or more and 400 um or less.

14. A cell transformation device, comprising:

   a pump unit, which is provided for pressurized supply of a cell solution and a material solution to a cell flow path and a material flow path of a cell transformation cartridge, respectively;
   a power application unit, which is provided to be connected to a terminal of the cell transformation cartridge to apply power to an electrode of the cell transformation cartridge; and
   a processor, which is electrically connected to the power application unit and the pump unit,

wherein the processor controls the pump unit based on the information of the cell transformation cartridge, a preset flow ratio, and a preset exposure time.

15. A cell transformation system, comprising:

a body unit, in which a cell flow path, a material flow path, and a resultant flow path that meet one another are formed;
an electric field forming unit, which comprises an electrode that is connected to the body unit and is placed at an inlet of the material flow path and an outlet of the resultant flow path;
a pump unit, which is provided for pressurized supply of a cell solution and a material solution to the cell flow path and the material flow path, respectively; and
a processor, which is electrically connected to the pump unit,
wherein the cell flow path and the material flow path are formed in a shape that converges to the resultant flow path,
wherein the resultant flow path comprises a mixing flow path extending from a location being connected to the cell flow path and the material flow path, and
the processor controls the pump unit such that the thickness of the solution being flowed into the mixing flow path through the cell flow path within the mixing flow path is smaller than the diameter of the cells contained in the cell solution.

16. A cell transformation method, comprising:

preparing a cartridge, in which a cell flow path, a material flow path, and a mixing flow path that meet one another are formed;
injecting a cell solution comprising cells into the cell flow path; and
injecting a material solution comprising a material for transformation of the cells into the material flow path,
wherein the ratio between the flow rate of the cell solution and the flow rate of the material solution being injected is a value that allows transformation to occur when the cells and the material meet in the mixing flow path.

17. The cell transformation method of claim 16, wherein the ratio is a value which allows the thickness, that is possessed within the mixing flow path by the solution being flowed into the mixing flow path through the cell flow path, to be less than three times the diameter of the cells.

18. The cell transformation method of claim 17, wherein the ratio is a value which allows the thickness, that is possessed within the mixing flow path by the solution being flowed into the mixing flow path through the cell flow path, to be less than the diameter of the cells.

19. The cell transformation method of claim 17, wherein the ratio is a value which allows the thickness, that is possessed within the mixing flow path by the solution being flowed into the mixing flow path through the cell flow path, to be smaller than the distance that the cells settle downward in the mixing flow path.

FIG.1

FIG.2

left

back ← → front

right

FIG.3

FIG.4

EP 4 600 342 A1

FIG.5

FIG.6

FIG.7

55d

52d

55d

542d

54d 541d

50d

55d

53d

27d

21d

24d

22d 23d 26d

40d

20d

25d

30d

10d

62d

up

front | right

left | back

down

1d

FIG.8

100

FIG.9

FIG.10

FIG.11

FIG.12

Culture media based eTX of eGFP-mRNA
(~0.78%, Neon, without applying R-buffer)

Culture media based eTX of eGFP-mRNA
(~98.3%, CellShot real-time transfection)

FIG.13

FIG.14

FIG.15

**(A)**

**(B)**

FIG.16

## INTERNATIONAL SEARCH REPORT

| International application No. |
|---|
| **PCT/KR2023/015094** |

### A. CLASSIFICATION OF SUBJECT MATTER

**C12M 1/42**(2006.01)i; **C12M 3/00**(2006.01)i; **C12M 1/00**(2006.01)i; **C12N 15/87**(2006.01)i

According to International Patent Classification (IPC) or to both national classification and IPC

### B. FIELDS SEARCHED

Minimum documentation searched (classification system followed by classification symbols)

C12M 1/42(2006.01); C12M 1/00(2006.01); C12N 1/00(2006.01); C12N 15/09(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 형질전환(transformation), 유로(flow path), 세포(cell), 전기장(electric field), 카트리지(cartridge)

### C. DOCUMENTS CONSIDERED TO BE RELEVANT

| Category* | Citation of document, with indication, where appropriate, of the relevant passages | Relevant to claim No. |
|---|---|---|
| A | KR 10-2020-0054885 A (FEMTOBIOMED INC.) 20 May 2020 (2020-05-20)<br>See claims 1-2 and 6; paragraphs [0015]-[0016] and [0036]; and figures 1a and 7-8. | 1-19 |
| A | KR 10-2004-0100932 A (FUJITSU LIMITED) 02 December 2004 (2004-12-02)<br>See entire document. | 1-19 |
| A | JP 2008-206397 A (TOHOKU UNIVERSITY) 11 September 2008 (2008-09-11)<br>See entire document. | 1-19 |
| A | KR 10-2021-0028687 A (NANOCAV, LLC) 12 March 2021 (2021-03-12)<br>See entire document. | 1-19 |
| A | KR 10-2020-0127546 A (LG CHEM, LTD.) 11 November 2020 (2020-11-11)<br>See entire document. | 1-19 |

☐ Further documents are listed in the continuation of Box C.          ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **15 January 2024** | **15 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/015094**

| Patent document cited in search report | | | Publication date (day/month/year) | Patent family member(s) | | | Publication date (day/month/year) |
|---|---|---|---|---|---|---|---|
| KR | 10-2020-0054885 | A | 20 May 2020 | CN | 112840015 | A | 25 May 2021 |
| | | | | EP | 3882332 | A1 | 22 September 2021 |
| | | | | WO | 2020-101254 | A1 | 22 May 2020 |
| KR | 10-2004-0100932 | A | 02 December 2004 | CN | 1572877 | A | 02 February 2005 |
| | | | | EP | 1479759 | A2 | 24 November 2004 |
| | | | | EP | 1479759 | A3 | 29 March 2006 |
| | | | | JP | 2004-344036 | A | 09 December 2004 |
| | | | | KR | 10-0636719 | B1 | 20 October 2006 |
| | | | | US | 2004-0235143 | A1 | 25 November 2004 |
| JP | 2008-206397 | A | 11 September 2008 | WO | 2006-123555 | A1 | 23 November 2006 |
| KR | 10-2021-0028687 | A | 12 March 2021 | AU | 2019-301062 | A1 | 04 February 2021 |
| | | | | AU | 2020-311908 | A1 | 11 February 2021 |
| | | | | AU | 2020-311908 | B2 | 23 February 2023 |
| | | | | CN | 112639112 | A | 09 April 2021 |
| | | | | DE | 112019003498 | T5 | 08 April 2021 |
| | | | | EP | 3818143 | A1 | 12 May 2021 |
| | | | | JP | 2021-535738 | A | 23 December 2021 |
| | | | | JP | 2023-018085 | A | 07 February 2023 |
| | | | | JP | 7185009 | B2 | 06 December 2022 |
| | | | | KR | 10-2546174 | B1 | 20 June 2023 |
| | | | | US | 11377652 | B2 | 05 July 2022 |
| | | | | US | 2020-0017847 | A1 | 16 January 2020 |
| | | | | US | 2020-0340014 | A1 | 29 October 2020 |
| | | | | US | 2021-0348155 | A1 | 11 November 2021 |
| | | | | US | 2022-0047862 | A1 | 17 February 2022 |
| | | | | WO | 2020-014264 | A1 | 16 January 2020 |
| | | | | WO | 2021-007315 | A1 | 14 January 2021 |
| KR | 10-2020-0127546 | A | 11 November 2020 | None | | | |

Form PCT/ISA/210 (patent family annex) (July 2022)